# EUROPEAN PATENT SPECIFICATION

(11) **EP 1 542 904 B1**
(45) Date of publication and mention of the grant of the patent: **16.01.2008**
(21) Application number: 03791745.7
(22) Date of filing: 26.08.2003
(51) Int. Cl.: B65B 31/00

(54) **PROCESS FOR PRODUCING METERED DOSE INHALER FORMULATIONS**
VERFAHREN ZUR HERSTELLUNG VON DOSIERINHALATORFORMULIERUNGEN
PROCEDE DE PRODUCTION DE PREPARATIONS POUR AEROSOLS-DOSEURS

(30) Priority: 27.08.2002 US 406127 P; 30.10.2002 US 422436 P
(43) Date of publication of application: 22.06.2005
(60) Divisional application: 07119781.8
(73) Proprietor: Schering Corporation, Kenilworth, New Jersey 07033-0530 (US)
(72) Inventor: CHAUDHRY, Saeed, Clifton, NJ 07011 (US); SHARPE, Stefan, A., Jersey City, NJ 07302 (US); BERRY, Julianne, Westfield, NJ 07090 (US); SEQUEIRA, Joel, A., Edison, NJ 08820 (US)
(74) Representative: Adams, Harvey Vaughan John
(86) International application number: PCT/US2003/026512
(87) International publication number: WO 2004/020289

(56) References cited:
- WO-A-00/51591
- WO-A-01/47493
- WO-A-02/11711
- WO-A-97/47286
- WO-A-98/08519
- WO-A-03/020253
- WO-A-03/086350
- US-A- 5 275 212
- US-A- 6 004 537

## Description

### BACKGROUND OF THE INVENTION

Metered dose inhalers have proven to be effective oral and nasal delivery systems that have been used extensively for delivering bronchodilating and steroidal compounds to asthmatics, as well as delivering other compounds such as pentamidine and non-bronchodilator anti-inflammatory drugs. The rapid onset of activity of compounds administered in this manner and the absence of any significant side effects have resulted in a large number of compounds being formulated for administration via this route. Typically, the drug is delivered to the patient by a propellant system generally comprising one or more propellants which have the appropriate vapor pressure and which are suitable for oral or nasal administration. The propellant systems typically comprised CFC propellant 11, CFC propellant 12, CFC propellant 114 or mixtures thereof. Often the vapor pressure of the propellant systems is adjusted by admixing a less volatile liquid excipient with the propellant.

However, propellants CFC 11, CFC 12 and CFC 114 belong to a class of compounds known as chlorofluorocarbons, which have been linked to the depletion of ozone in the atmosphere. It has been postulated that ozone blocks certain harmful UV rays and thus a decrease in the atmospheric ozone content will result in an increase in the incidence of skin cancer. In the 1970's certain steps were taken to reduce the CFC emissions from aerosols. Other propellants, such as hydrocarbons, were used, or the product was delivered in a different manner. Because CFC usage in medicinal applications is relatively low i.e. less than 1 % of total CFC emissions, and because of the health benefits associated with metered dose inhalers, steps were not taken at that time to restrict the use of CFC propellants in metered dose inhalers.

However, continuing and more sophisticated ozone measurements have indicated that the earlier restrictions in CFC usage were insufficient and that additional, significant steps should be taken to drastically reduce CFC emissions. Recommendations have been made that CFC production be virtually discontinued. As a result, it may not be possible to continue to use CFC propellants in the intermediate and long term. While some efforts have been made to use non-pressurized metered dose inhalers, many of these devices have not been completely successful. Some of the performance issues related to these are: delivery of uniform doses, mechanical complexity, ability to provide the required doses per unit of an aerosol container, ability to meet stringent regulatory standards, the inhalers can be difficult for individuals to utilize, and are bulky and/or cumbersome for the patients to use, particularly when patients have an acute need for the medication.

As a result, there is a need for pressurized aerosol formulations, such as metered dose inhalers, which are substantially free of CFC's. Non-CFC propellants systems must meet several criteria for pressurized metered dose inhalers. They must be non-toxic, stable and non-reactive with the medicament and the other major components in the valve/actuator. One propellant which has been found to be suitable is CF₃CHFCF₃, also known as HFA 227, HFC 227 or 1,1,1,2,3,3,3 heptafluoropropane. Another such propellant for use in metered dose inhalers is CF₃CH₂F, also known as 1,1,1,2-tetrafluoroethane or HFA 134a.

WO 98/08515, US 6004537, WO 02/11711 and WO 97/47 286 disclose a solution aerosol formulation adapted for use in a pressurized aerosol container free of CFC's.

Filling processes of the canister used in metered dose inhalers that contain the medication typically employ a two stage process for filling canister. In one such process, the drug, surfactant, i.e., oleic acid and the non-volatile excipient, i.e., ethanol, are filled into the container in the first stage, and the propellant is added in to the container in the second stage. For CFC propellant formulations, typically a surfactant is mixed with a less volatile propellant (i.e. P-11) and then there is added a more volatile propellant through the valve.

Mometasone MDI-AP has previously been manufactured using such a conventional pressure filling process. This process is a two-stage process. In the first stage the drug is mixed in with oleic acid and alcohol to form a homogenous well-dispersed suspension which is typically called the "concentrate." The required amount of concentrate is filled into an open can. In the second stage the valve is then crimped onto the can and the propellant (HFA 227) is filled into the can through the valve. A significant disadvantage of this formulation is that this two-stage manufacturing process resulted in particle size growth of the drug while the concentrate was being filled during a typical manufacturing and filling operation. A fine particle size is essential for delivery of aerosolized suspensions to the deeper passages of the lungs. In addition, this is a less accurate fill method. Drug concentrate typically comprises less than 5% of the formulation and problems can arise with regards to the accurate filling at these low concentrations.

However, the specific combinations noted above may not provide the desired solubility, stability, low toxicity, exact dosage, correct particle size (if suspension) and/or compatibility with commonly used valve assemblies of metered dose inhalers. Accordingly, there exists a need for CFC free formulations for the treatment of asthma, and processes for producing the same, that do not suffer from the aforementioned infirmities.

### SUMMARY OF THE INVENTION

Accordingly, the present invention is directed to process for introducing a suspension or solution of mometasone furoate anhydrous into a metered dose inhaler container according to claim 1. The present invention also is directed to the product produced by the aforementioned method.

There is also disclosed a process for introducing a suspension or solution of mometasone furoate anhydrous into a metered dose inhaler container, said container having a valve attached thereto, said method comprising the steps of: a) introducing mometasone furoate anhydrous, a surfactant and a chlorflourocarbon free propellant into a vessel that is held under pressure to form a suspension or solution, wherein said pressure is greater than about 30 psi; b) circulating said suspension or solution from the vessel through a line which includes a filling head and a double diaphragm pump; c) bringing said filling head into communication with said metered dose inhaler container through said valve of said metered dose inhaler container; d) introducing a quantity of such suspension or solution into the container from the filling head of the line through said valve of said metered dose inhaler container; e) withdrawing said filling head from said metered dose inhaler container; and f) sealing said metered dose inhaler container.

Also disclosed is a process for introducing a process for introducing a suspension or solution of a compound selected from the group consisting of mometasone furoate anhydrous, formoterol fumarate and combinations thereof, into a metered dose inhaler container, said container having a valve attached thereto, said method comprising the steps of: a) introducing mometasone furoate anhydrous, formoterol fumarate and combinations thereof, a surfactant and a chlorflourocarbon free propellant into a vessel that is held under pressure to form a suspension or solution, wherein said pressure is about 10 psi to about 15 psi; b) circulating said suspension or solution from the vessel through a line which includes a filling head and a double diaphragm pump; c) bringing said filling head into communication with said metered dose inhaler container through said valve of said metered dose inhaler container; d) introducing a quantity of such suspension or solution into the container from the filling head of the line through said valve of said metered dose inhaler container; e) withdrawing said filling head from said metered dose inhaler container; and f) sealing said metered dose inhaler container.

Also disclosed is process for introducing a suspension or solution of mometasone furoate anhydrous into a metered dose inhaler container, said container having a valve attached thereto, said method comprising the steps of: a) introducing mometasone furoate anhydrous, a surfactant and a chlorfluorocarbon free propellant into a vessel that is held under pressure to form a suspension or solution, wherein said pressure is greater about 0 psi to about 10 psi; b) circulating said suspension or solution from the vessel through a line which includes a filling head and a single diaphragm pump; c) bringing said filling head into communication with said metered dose inhaler container through said valve of said metered dose inhaler container; d) introducing a quantity of such suspension or solution into the container from the filling head of the line through said valve of said metered dose inhaler container; e) withdrawing said filling head from said metered dose inhaler container; and f) sealing said metered dose inhaler container as well as the products produced thereby.

### DETAILED DESCRIPTION OF THE INVENTION

According to the present invention the metered dose inhaler formulation is manufactured utilizing a new process in which the particle size growth of the drug on manufacturing/filling is eliminated.

In one embodiment of the invention, this new process, the entire formulation drug/oleic acid/alcohol/HFA 227 is mixed in a single compounding vessel which is covered by a lid under pressure. This pressurized vessel allows for the gaseous propellant to exist in the liquid state. Next, the formulation in liquid form (some of the HFA 227 may be in the gaseous state) is filled in one-stage through the valve of an empty metered dose inhaler canister containing a previously crimped on valve.

In another embodiment of the invention, this new process also resulted in markedly reducing particle size growth of drug in metered dose inhaler formulation, as compared to the particle size growth seen with the conventional two-stage manufacturing process.

This new process provides improved product quality due to better fill tolerance associated with the new process. Additionally, the new process also results in operational improvements such as a) one filling station, b) no need for concentrate and neat propellant sample checks.

Most preferably, in accordance with the present invention, the dosing systems containing at least one pharmacologically active agent or drug is a material capable of being administered to the respiratory system, including the lungs. For example, a drug in accordance with the present invention could be administered so that it is absorbed into the blood stream through the lungs.

A particularly preferred corticosteroid is mometasone furoate, the active component of ELOCON® lotion, cream, and ointment, and NASONEX® nasal spray, that is an anti-inflammatory corticosteroid having the chemical name, 9,21-Dichloro-11 (beta),17-dihydroxy-16(alpha)-methylpregna-1,4-diene-3,20-dione 17-(2 furoate). Mometasone furoate is a preferred active ingredient, although other active ingredients and/or combinations thereof may be used within the scope of the present invention. Mometasone furoate is a white powder, with an empirical formula of C₂₇ H₃₀C₁₂O₆. It is practically insoluble in water; slightly soluble in methanol, ethanol, and isopropanol; soluble in acetone and chloroform; and freely soluble in tetrahydrofuran. Mometasone can exist in various hydrated, crystalline and enantiomeric forms, e.g., as a monohydrate. This product is available from Schering-Plough Corporation, Kenilworth, New Jersey.

Also prefererred pharmacologically active agents in accordance with the present invention include, for example, Formoterol (also known as eFormoterol) e.g., as the fumarate or tartrate, a highly selective long-lasting β₂-adrenergic agonist having bronchospasmolytic effect, is effective in the treatment of reversible obstructive lung ailments of various genesis, particularly asthmatic conditions.

Another particularly preferred β-agonist is albuterol sulfate. The active component of PROVENTIL® HFA (albuterol sulfate) Inhalation Aerosol is albuterol sulfate, USP racemic (alpha) ¹ [(tert -Butylamino)methyl]-4-hydroxy- m -xylene-(alpha),(alpha)'-diol sulfate (2:1)(salt), a relatively selective beta ₂-adrenergic bronchodilator. Albuterol sulfate is the official generic name in the United States. The World Health Organization recommended name for the drug is salbutamol sulfate. Albuterol sulfate is a white to off-white crystalline solid. It is soluble in water and slightly soluble in ethanol. PROVENTIL® HFA Inhalation Aerosol is a pressurized metered-dose aerosol unit for oral inhalation. It contains a microcrystalline suspension of albuterol sulfate in propellant HFA-134a (1,1,1,2-tetrafluoroethane), ethanol, and oleic acid. PROVENTIL® HFA is available from Schering-Plough Corp., Kenilworth, New Jersey.

Several of these compounds could be administered in the form of pharmacologically acceptable esters, salts, solvates, such as hydrates, or solvates of such esters or salts, if any. The term is also meant to cover both racemic mixtures as well as one or more optical isomers.

It is preferred that the formulation be a CFC-free pressurized aerosol formulation. Non-CFC propellants systems must meet several criteria for pressurized metered dose inhalers. They must be non-toxic, stable and non-reactive with the medicament and the other major components in the valve/actuator. One propellant which has been found to be suitable for use in the present invention is CF₃ CHFCF₃, also known as HFA 227, HFC 227 or 1,1,1,2,3,3,3 heptafluoropropane, hereinafter HFA 227. Another such propellant for use in the present invention is CF₃CH₂F, also known as 1,1,1,2-tetrafluoroethane or HFA 134a, hereinafter HFA 134a.

In formulations of the present invention which are suitable for treating lower respiratory system disorders such as asthma, at least a substantial portion of the drug is present as suspended particles having respirable sizes, e.g., about 0.5 to about 10 micrometers in their largest dimension. In formulations which are suitable for treating upper respiratory system disorders such as rhinitis, somewhat larger drug particles may be permissible, but the foregoing size range remains preferred.

The processes for producing the formulations of the present invention utilize propellants HFA 227 or HFA 134a, or a combination thereof, in combination with a pharmacologically active agent, preferably but not limited to mometasone furoate anhydrous, optionally, a liquid excipient, and optionally a surfactant. The excipient facilitates the compatibility of the medicament with the propellant and also lowers the discharge pressure to an acceptable range, i.e., about 2.76-5.52 X 10⁵ newton/meter² absolute (40 to 80 psi), preferably 3.45-4.83 X 10⁵ newton/meter² absolute (50 to 70 psi). The excipient chosen must be non-reactive with the medicaments, relatively non-toxic, and should have a vapor pressure below about 3.45 X 10⁵ newton/meter² absolute (50 psi).

As used hereinafter the term "medium chain fatty acids" refers to chains of alkyl groups terminating in a -COOH group and having 6-12 carbon atoms, preferably 8-10 carbon atoms. The term "short chain fatty acids" refers to chains of alkyl groups terminating in a -COOH group and having 4-8 carbon atoms. The term "alcohol" includes C₁-C₃ alcohols, such as methanol, ethanol and isopropanol.

Among the preferred excipients are: propylene glycol diesters of medium chain fatty acids available under the tradename Miglyol 840 (from Huls America, Inc. Piscataway, N.J.); triglyceride esters of medium chain fatty adds available under the tradename Miglyol 812 (from Huls); perfluorodimethylcyclobutane available under the tradename Vertrel 245 (from E. I. DuPont de Nemours and Co. Inc. Wilmington, Del.); perfluorocyclobutane available under the tradename octafluorocyclobutane (from PCR Gainsville, Fla.); polyethylene glycol available under the tradename EG 400 (from BASF Parsippany, N.J.); menthol (from Pluess-Stauffer International Stanford, Conn.); propylene glycol monolaurate available under the tradename lauroglycol (from Gattefosse Elmsford, N.Y.); diethylene glycol monoethylether available under the tradename Transcutol (from Gattefosse); polyglycolized glyceride of medium chain fatty adds available under the tradename Labrafac Hydro WL 1219 (from Gattefosse); alcohols, such as ethanol, methanol and isopropanol; eucalyptus oil available (from Pluses-Stauffer International); and mixtures thereof.

A surfactant optionally may be added to lower the surface and interfacial tension between the medicaments and the propellant. Where the medicaments, propellant and excipient are to form a suspension, a surfactant may or may not be required. Where the medicament, propellant and excipient are to form a solution, a surfactant may or may not be necessary, depending in part, on the solubility of the particular medicament and excipient. The surfactant may be any suitable, non-toxic compound which is non-reactive with the medicament and which substantially reduces the surface tension between the medicament, the excipient and the propellant and/or acts as a valve lubricant.

A surfactant may also frequently included in aerosol formulations, for purposes such as assisting with maintaining a stable suspension of the drug and lubricating the metering valve. The formulation of the present invention does not require a surfactant for maintenance of ready dispersability (such as by moderate agitation immediately prior to use), as the drug forms loose floccules in the formulation and does not exhibit a tendency to settle or cream when alcohol is present. Upon undisturbed storage, the drug particles remain suspended in their flocculated state when alcohol is present.

Among the preferred surfactants are: oleic acid available under the tradename oleic acid NF6321 (from Henkel Corp. Emery Group, Cincinnati, Ohio); cetylpyridinium chloride (from Arrow Chemical, Inc. Westwood, N.J.); soya lecithin available under the tradename Epikuron 200 (from Lucas Meyer Decatur, III.); polyoxyethylene(20) sorbitan monolaurate available under the tradename Tween 20 (from ICI Specialty Chemicals, Wilmington, Del.); polyoxyethylene(20) sorbitan monostearate available under the tradename Tween 60 (from ICI); polyoxyethylene(20) sorbitan monooleate available under the tradename Tween 80 (from ICI); polyoxyethylene (10) stearyl ether available under the tradename Brij 76 (from ICI); polyoxyethylene (2) oleyl ether available under the tradename Brij 92 (frown ICI); Polyoxyethylene-polyoxypropylene-ethylenediamine block copolymer available under the tradename Tetronic 150 R1 (from BASF); polyoxypropylene-polyoxyethylene block copolymers available under the tradenames Pluronic L-92, Pluronic L-121 end Pluronic F 68 (from BASF); castor oil ethoxylate available under the tradename Alkasurf CO-40 (from Rhone-Poulenc Mississauga Ontario,Canada); and mixtures thereof.

Mometasone furoate is slightly soluble in ethanol. As with other drugs which have solubility in ethanol, there is a tendency for mometasone furoate to exhibit crystal growth in ethanol-containing formulations. Formulation parameters which do not promote drug particle size growth are known. These parameters provide the advantage of minimizing the required ethanol concentrations, to reduce the potential for unpleasant taste sensations and render the compositions more suitable for use by children and others with low alcohol tolerance. A certain minimum level of ethanol is preferred to provide consistent and predictable delivery of the drug from a metered dose dispenser. This minimum level is about 1 weight percent of the total formulation, which results in a marginally acceptable drug delivery. Increased amounts of ethanol generally improve drug delivery characteristics.

However, and to prevent drug crystal growth in the formulation, it is preferred to limit the concentration of ethanol. Experimental data indicate that the ratio of the weight of mometasone furoate to the weight of ethanol is important in preventing particle size increases.

The available metering valve delivery volumes range from about 25 to about 100 microliters per actuation, while the amounts of drug substance required in a dose for treating a particular condition is generally about 10 to about 500 micrograms per valve actuation. These two factors combined pose limitations that dictate the points within the foregoing ethanol parameters for a given formulation. The determination of such amounts is within the skill of workers in this art.

Where the active compound forms a suspension, the particle size should be relatively uniform, with substantially all the particles preferably ranging between about 0.1-25 microns, preferably 0.5-10 microns, more preferably 1-5 microns. Particles larger than 25 microns may be held up in the oropharyngeal cavity, while particles smaller than about 0.5 micron preferably are not utilized, since they would be more likely to be exhaled and, therefore, not reach the lungs of the patient.

The formulations of the present invention may be filled into the aerosol containers using conventional filling equipment. Since propellants 227 and 134 may not be compatible with all elastomeric compounds currently utilized in present aerosol valve assemblies, it may be necessary to substitute other materials, such as white buna rubber, or to utilize excipients and optionally surfactants which mitigate the adverse effects of propellant 227 or 134 on the valve components.

Depending on the particular application, the container may be charged with a predetermined quantity of formulation for single or multiple dosing. Typically, the container is sized for multiple-dosing, and, therefore it is very important that the formulation delivered is substantially uniform for each dosing. For example, where the formulation is for bronchodilation, the container typically is charged with a sufficient quantity of the formulation for 200 charges.

Suitable suspensions may be screened in part by observing several physical properties of the formulation, i.e. the rate of particle agglomeration, the size of the agglomerates and the rate of particulate creaming/settling and comparing these to an acceptable standard. Such, suitable solutions may be screened/evaluated by measuring the solubility of the medicament over the entire recommended storage temperature range.

Suspensions of the present invention preferably may be prepared by either the pressure filling or cold filling procedures known in the art. For metered dose inhalers, suspensions may be particularly preferred for efficacy and stability considerations.

Those skilled in the art may choose to add one or more preservative, buffer, antioxidant, sweetener and/or flavors or other taste masking agents depending upon the characteristics of the formulation.

The processes and products produced thereby of the present invention overcome stability problems i.e. crystal growth during compounding/filling encountered while formulating. The addition of the propellant in a single stage quenches crystal growth of the pharmaceutical active, particularly with mometasone furoate anhydrous since the solubility in the concentrate is much greater than that in the final formulation. Suspensions in which a high proportion of drug is dissolved have a tendency to exhibit particle size growth by a phenomena known as Ostwald Ripening.

Further, the process itself is very robust process and reproducible. The particle size distribution of the product for each batch is reproducible and the fill weight of each ingredient is the same in each canister. Indeed, the particle size of the active is maintained throughout the process, which is very important as the final product is to be administered to patients and uniformity of particle size below 10 microns for inhalation is extremely important. Another added advantage is that of excellent process capability (0.98 correlation coefficient) for PSD as is demonstrated by a good linear relationship of drug substance particle size to drug product particle size. And, it conforms to stringent FDA requirements for both batch to batch and unit to unit within the batch content uniformity, reproducibility, particle size distribution, and drug content uniformity.

This invention further relates to the improvement in the quality with regards to both particle size uniformity and formulation stability of the mometasone furoate MDI Oral and Nasal Suspension, either alone or combined with other drug substances, e.g. formoterol fumarate, by controlling the particle size of the suspended mometasone furoate drug substance. For mometasone furoate MDI, it has been found that the quality of the drug product is linked to the particle size range of the suspended drug substance. There is a rank order correlation of the quality of the product with a decrease in the size range of the corresponding drug substance suspended in the product. It was determined that drug substance containing a high proportion of large crystals > having a particle size of greater than 10 microns produces a product with unacceptable particle growth with time and temperature. However, it has been found that when the particle size of the drug substance is less tahn 10 microns, a product is produced which has uniform suspended drug particles with a markedly, surprising improved and stable particle size profile with time and temperature.

In the case of the oral MDI containing mometasone furoate, an example of an acceptable product profile for the 100 microgram per actuation strength, using an Anderson cascade impactor and 1-liter entry port, is given below (for 2 actuations dose):
Group 1 - Entry port + Stage 0 = 9-14 µg
Group 2 - Stage 1 + Stage 2 = 18-19 µg
Group 3 - Stage 3 + Stage 4 = 131-132 µg
Group 4 - Stage 5-Filter = 26-27µg
Group 5 - Total Drug Recovery = 194-198 µg
% Fine Particles (Stages 3-Filter) = 79-82 %

The size of the suspended mometasone furoate drug contained in the drug product may be controlled in the following ways. First, the drug substance may be more efficiently milled prior to product batch manufacture. This could include reducing the micronization feed rate, employing centrifugal classification to remove larger particles and increasing the number of cycles that the material is fed into the micronizer (i.e., double micronizing). Alternatively, The drug substance may be spray dried prior to product batch manufacture (i.e., including super critical fluid technology) to create uniformly small drug substance particles. Also, the method of manufacture can be modified by various methods, i.e., by reducing the temperature of batch manufacture, reducing the level of alcohol used to prepare the drug concentrate, and/or reducing the homogenization time. Finally, other processes of controlling drug substance particle size known in the art, e.g., using surfactants or other particle size growth retardation approaches. This aspect of the invention is not compound specific and does not solely relate to Mometasone Furoate. It also applies to other systems in which a material or materials are suspended in a liquid medium.

The foregoing descriptions of various embodiments of the invention are representative of various aspects of the invention, and are not intended to be exhaustive or limiting to the precise forms disclosed. Many modifications and variations undoubtedly will occur to those having skill in the art. It is intended that the scope of the invention shall be fully defined solely by the appended claims.

### Example 1

The compounding area should preferably be at a room temperature of <70°F and a humidity level of ≤60% RH. The compounding tank should be pressure rated to a minimum of 100 psig and should contain a safety valve that will allow for slow release of contents if the pressure exceeds 100 psig.

Charge about 90% of the alcohol into a suitable premix vessel. Cool the contents of the premix vessel to 0 ± 5°C. Charge the oleic acid into the premix vessel with homogenization (Silverson, in-dwelling). Rinse the weighing container with the remainder (about 10%) of the alcohol and add the rinses to the premix vessel with homogenization. Homogenize for approximately 5 minutes or until the oleic acid has completely dissolved.

Charge the micronized mometasone furoate anhydrous, into the premix vessel and homogenize (Silverson, in-dwelling) at high speed for approximately 5 minutes or until a uniform and smooth suspension has formed. Determine the weight of mometasone furote anhydrous left in the container after charging it to the batch. If that weight is greater than 0.5% of the theoretical active batch charge, then add additional active (equivalent to the amount of left on the container) to the batch.

Next, charge the contents of the premix vessel into the compounding tank. Completely seal the compounding tank. Charge to the sealed tank the HFA 227 that has been filtered through an integrity tested filter, e.g., Aervent cartridge filter from Millipore with teflon encapsulated O-rings.

Begin agitation with a suitable mixer (propeller mixer) and continue for the duration of the filling operation. Begin circulation of the product through the aerosol-filling machine (Pamasol) and maintain the temperature of the compounding tank at refrigeration (approximately 0±10°C) for the duration of the filling operation.

Fit and crimp the valve onto the can. The valve crimp height guideline should be 5.7 ± 0.1 mm (measured by a Socoge gage). The valve crimp diameter guideline is 17.7 ± 0.1 mm (measured by a micrometer). The ranges may vary as is known to one of skill in the art. Measure periodically and make the necessary adjustments to achieve the recommended guideline values. Meter the required weight of product into each can. Check the fill weight of the product periodically and make the necessary adjustments to achieve the in-process control limits.

Leak test cans upon completion of batch manufacture (at least 1 can in 500) by immersing in a 55-60°C water bath for 5-10 minutes. The samples that are leak tested should not show leakage or permanent deformation. Discard any part of the batch in which leakage or permanent deformation is observed in this leak test. The leak test was in accordance with 49 CFR 173.306(b) (3)).

Lagger cans for a minimum of 10 days. Prior to spray testing. Upon completion of laggering, spray test (minimum 3 actuations/can) and check weigh cans according to procedures known in the art.

### Example 2

Initially, it was determined that a minimum of 30 psi pressure must be maintained inside the product compounding vessel in order to meet the product fill weight requirements. This pressure is needed for the proper operation of the dual piston Pamasol pump, which transfers the product from the compounding tank to the Pamasol filling machine.

The 30 psi pressure in the system can be obtained by increasing the temperature of the product vessel to >10°C. This results in evaporation of propellant (i.e., HFA-227) into the headspace of the mixing vessel which becomes more significant during the course of filling operation, i.e., as the headspace in the vessel increases due to the removal of product. Thus, as the propellant concentration decreases, the concentration of the other ingredients in the product correspondingly increase, leading to levels that cannot meet the product specifications.

In another embodiment of the present invention, one can re-charge propellant into the batch at the point where evaporation is significant enough to affect product quality. This approach adds an additional step to batch manufacture, but also may lead to a less robust process since the levels added are difficult to control and are a time consuming process.

An alternate means of having 30 psi pressure in the vessel while, at the same time, allowing the temperature to be low enough to avoid evaporation of propellant, is to charge high pressure gases i.e., nitrogen, compressed air, etc., into the product compounding vessel. This approach has many drawbacks as well which include the introduction of increased levels of water in the product, drug degradation for air and moisture sensitive compounds and formation of pockets of trapped gases within the liquid (i.e., air bubbles) which can lead to erratic dose delivery.

This invention, is a substantial improvement to the current Pamasol one-step filling operation, in that it allows the product to be filled reproducibly at low pressure (<< 30 psi) and thus resolves the above issues. In one aspect of the invention, there is the use of a single or dual pump system for the filling of areosol formulations from the compounding tank while maintaining recirculation through the filling lines. These pumps function as follows: A single pump is used when the pressure in the tank is 0 to 10 psi. This pump is available as a Versa-Matic Double Diaphragm pump. An alternate pump is the Versa-Matic Double Diaphragm pump or the Pamasol Double Diaphragm pump for when the pressure is greater than 10 psi yet lower than 15 psi. The sequence of the pumps and the tank in this dual system is critical and should be in the following order. Compounding tank then Versa-Matic Double Diaphragm pump, Pamasol Double Diaphragm pump and finally the Pamasol filler. This aspect of the invention is dictated by the pressure maintaining capability of the two pumps and the pressure requirement for the Pamasol filler.

Both pump systems, either individually or combined, e.g., the single pump system utilizing the Versa-Matic Double Diaphragm pump, as well as the dual pump system utilizing the Versa-Matic Double Diaphragm Pump and the Pamasol Double Diaphragm pump maintain the pressure greater than 30 psi. This pressure is required by the Pamasol filling machine for reproducible filling in to the aerosol cans as well as for proper recirculation through the filling lines.

With this new system, the product fill weight requirement is met and the batch yield is > 90%.

Accordingly, in one aspect of the present invention, the following procedure has been carried out. Initially, flush the compounding vessel, the dual piston pump, the Pamasol filler and all lines with Nitrogen for about 15 minutes. Thereafter, evacuate all of the gasses by vacuum. Activate the chiller and set the temperature for about -20 degrees Celsius. Compound an aerosol batch by charging all of the ingredients into a completely sealed aerosol compounding vessel. Begin mixing the ingredients while cooling the product. Open the bottom valve of the compounding vessel and start re-circulating the product through the dual piston pump back to the compounding vessel until the pressure of the compounding vessel is less than or equal to about 5 psi. Stop the pump, and disconnect the return line of the vessel. Connect this line to the inlet of the Pamasol filler and outlet of the pamasol filler connect to the inlet of the compounding vessel. Start re-circulation of the product until the vapor pressure reaches less than or equal to about 3 psi. Fill the 15 ml aerosol cannisters with a fill weight of about 16 g +/- 0.3 grams. Check the weight of 10 cans from the beginning, middle and end of the batch.

The following results were obtained as set forth below.

**Table 1**

| **Can Net Fill Weight of the Product** | | | |
|---|---|---|---|
| **Can Number** | **Beginning of the Batch (g)** | **Middle of the Batch (g)** | **End of the Batch (g)** |
| 1 | 16.23 | 16.15 | 16.09 |
| 2 | 16.17 | 16.11 | 16.09 |
| 3 | 16.13 | 16.13 | 16.13 |
| 4 | 16.16 | 16.13 | 16.12 |
| 5 | 16.13 | 16.14 | 16.11 |
| 6 | 16.17 | 16.10 | 16.09 |
| 7 | 16.14 | 16.14 | 16.10 |
| 8 | 16.16 | 16.11 | 16.11 |
| 9 | 16.17 | 16.11 | 16.11 |
| 10 | 16.16 | 16.13 | 16.12 |
| Average | 16.16 | 16.13 | 16.11 |

Throughout the fill process, the in beginning of the batch the compound vessel was operated at 3 psi and a temperature of -11.1 degrees Celsius, in the middle of the process the compound vessel was operated at 0.5 psi and a temperature of -12.5 degrees Celsius, and in the end of the process the compound vessel was operated at 0 psi and a temperature of -13.3 degrees Celsius.

### Example 3

Another aspect of the present invention is defined as a novel method of one-step filling and manufacture/compounding of a dispersion system of a well mixed suspension, e.g. mometasone furoate in ethanol/oleic acid suspension medium to which small amounts of the propellant e.g. HFA-227, HFA-134a, CFC 11. 12, 114, are added continuously to a final weight in a pressurized compounding vessel.

The basis of the invention is the continuous addition of vaporized or liquid propellant to compensate and prevent the gradual loss of propellant from the liquid phase to the vapor phase, i.e., evaporation. This evaporation occurs in the course of filling and leads to a gradual increase of the concentration of the active drug substance, mometasone furoate, in the finished product. This loss of propellant is driven by the fact that the composition of liquid and vapor phases is not the same. The vapor becomes richer in the more volatile component (propellant) and therefore, the mole fraction of this more volatile component is higher in the vapor phase while the mole fraction of the propellant in the liquid phase decreases thus increasing the concentration of the active drug substance and the ethanol/oleic acid mixture. The propellant loss leads to a decrease in the final yield of the finished product (up to approximately 30% loss due to unfilled end portions of the suspension). This may require maintaining very low filling temperatures; i.e. cold filling (to reduce evaporation) which leads to added technological and processing difficulties.

The following approaches can be applied for propellant addition to compensate for evaporative losses: (1) the propellant is maintained at approximately 30°C and added continuously (as a vapor) to the compounding vessel at a constant rate; (2) the propellant vapor generation can also be achieved by transporting the liquid propellant to a holding depressurized vessel where the liquid propellant is allowed to expand and thus evaporate. The depressurized propellant vapor can then be added to the suspension compounding vessel as specified above; finally (3) the propellant can also be added as a liquid throughout the filling process of the batch manufacture.

All three approaches result in a suspension that is metered with the Pamasol filling equipment into the individual aerosol cans which were previously crimped with appropriate valves.

The advantages of this method of manufacture by continuous addition of propellant are the following: (1) the manufacture of MDI (Metered Dose Inhaler) with minimal evaporative losses; (2) a product that exhibits consistent Drug Content Uniformity ("DCU") throughout the filling process; (3) ease of manufacture, e.g. the need for excessive sampling and DCU testing is obviated; and (4) higher yield of the finished product - up to 30% extra finished product can be filled.

Accordingly, prepare an aerosol batch in a chilled and sealed compounding tank at a temperature of greater than about 5 degrees Celsius. Fill the vaporized propellant vessel with propellant to about 25% of its capacity, weigh and then heat the vessel to 30 degrees Celsius to vaporize the propellant at a pressure of about 70 psi. Connect the vapor terminal of the vaporized propellant vessel to the batch compounding vessel. Open the valve at the vapor terminal of the vaporized propellant vessel. Open and adjust the regulator valve of the compounding vessel to maintain 30 to 40 psi pressure during the filling operation. Fill the aerosol product on to the 15 ml cannisters. Check the fill weight of the filled cans.

**Table 2**

| **Can Net Fill Weight of the Product** | | | |
|---|---|---|---|
| **Can Number** | **Beginning of the Batch (g)** | **Middle of the Batch (g)** | **End of the Batch (g)** |
| 1 | 16.01 | 16.01 | 16.01 |
| 2 | 16.01 | 16.01 | 16.00 |
| 3 | 16.02 | 16.02 | 16.01 |
| 4 | 16.01 | 16.01 | 16.01 |
| 5 | 16.01 | 16.02 | 16.01 |
| 6 | 16.01 | 16.03 | 16.02 |
| 7 | 16.02 | 16.00 | 16.02 |
| 8 | 16.01 | 16.01 | 16.02 |
| 9 | 16.01 | 16.01 | 16.01 |
| 10 | 16.01 | 16.01 | 16.01 |
| Average | 16.01 | 16.01 | 16.01 |

Further evidence for the absence of HFA-227 evaporation during filling due to the addition of the propellant vapor are the Drug Content Uniformity results obtained from the beginning and the end of the filling run, e.g., about 90 %, preferably about 92%

Next, perform cascade impactor tests using the metered dose inhalers produced above, preferably in accordance with USP reference standards. Cascade Impactor tests were performed utilizing the Anderson Cascade Impactor with a 1-liter entry port as is known to one of skill in the art. This assay demonstrates that as the drug substance median increases, the drug product particle size increases. The correlation of drug substance median with the percentage of fine particles of product is 0.98. The Andersen Cascade Impactor is widely used for measuring the particle size distribution of airborne particles and more specifically pharmaceutical aerosols. The eight stage Andersen Impactor separates the sample into nine size intervals when used with a backup filter after the last impaction stage. The fine particle fraction is defined as the percentage of particles having a particle size of less than 4.7 µm. The fine particle dose is defined as the amount in µg per dose that is less than 4.7 µm in size in each actuation. The µg/shot is the total amount of emitted drug product that exits the metered dose inhaler upon actuation. The particle size distribution of the powder is characterized by mass median aerodynamic diameter (MMAD).

**Table 3**

| The following table describes the Andersen Cascade Impactor Results for a 100 µg mometasone furoate per actuation product produced in accordance with the process of the present invention. | | |
|---|---|---|
| **Drug Substance Median, µm** | **Drug Product MMAD, µm** | **Drug Product % Fine Particles** |
| 1.14 | 2.58 | 79.2 |
| 1.19 | 2.63 | 75.6 |
| 1.25 | 2.83 | 68.1 |
| 1.38 | 3.09 | 56.8 |
| 1.53 | 3.54 | 50.3 |
| 1.77 | 4.38 | 37.6 |

**Table 4**

| The following table describes the Andersen Cascade Impactor Results for a 200 µg mometasone furoate per actuation product produced in accordance with the process of the present invention. | | |
|---|---|---|
| **Drug Substance Median, µm** | **Drug Product MMAD, µm** | **Drug Product % Fine Particles** |
| 1.14 | 2.79 | 76.8 |
| 1.29 | 3.42 | 57.6 |

**Table 5**

| The following table describes the Andersen Cascade Impactor Results for a 100 µg mometasone furoate per actuation product produced by a two stage process. | | | | |
|---|---|---|---|---|
| **Cascade Impactor Stage or Accessory** | **Upper Particle Size Limit µm** | **% Drug Recovery During Batch Filling** | | |
| | | **0 hours** | **3.5 hours** | **24 hours** |
| 0 | 10 | 1.52 ± 0.03 | 1.60 ± 0.01 | 3.26 ± 0.36 |
| 1 | 9.0 | 8.38 ± 0.36 | 8.79 ± 0.14 | 19.4 ± 1.09 |
| 2 | 5.8 | 12.9 ± 0.50 | 11.8 ± 2.93 | 18.1 ± 0.43 |
| 3 | 4.7 | 34.2 ± 0.47 | 32.0 ± 1.92 | 25.9 ± 0.25 |
| 4 | 3.3 | 17.0 ± 0.24 | 14.5 ± 0.96 | 6.98 ± 0.17 |
| 5 | 2.1 | 5.05 ± 0.45 | 4.49 ± 0.24 | 3.59 ± 0.19 |
| 6 | 1.1 | 2.04 ± 0.17 | 2.00 ± 0.12 | 1.77 ± 0.16 |
| 7 | 0.65 | 0.88 ± 0.13 | 0.93 ± 0.08 | 0.82 ± 0.13 |
| F | 0.43 | 1.17 ± 0.29 | 1.19 ± 0.09 | 1.03 ± 0.26 |
| Entry Port | n/a | 12.7 ± 1.21 | 16.0 ± 5.00 | 14.8 ± 1.86 |
| Casings | n/a | 4.16 ± 0.45 | 4.35 ± 0.32 | 5.03 ± 0.41 |

As is evident from a comparison of the particle sizes over time during the filling process, there is a significant variation in the particle size in the early stages of the process relative to the particle size at the end of the filling run. It is very important for the particle size to remain consistent during processing.

**Table 6**

| The following table describes the Andersen Cascade Impactor with a 1 liter entry port Results for a 100 µg mometasone furoate per actuation product produced in accordance with the process of the present invention. | | | | |
|---|---|---|---|---|
| **Cascade Impactor Stage or Accessory** | **Upper Particle Size Limit µm** | **% Drug Recovery During Batch Filling** | | |
| | | **0 hours** | **24 hours** | **48 hours** |
| 0 | 10 | 1.12 ± 0.11 | 1.32 ± 0.30 | 1.28 ± 0.08 |
| 1 | 9.0 | 5.56 ± 0.09 | 6.45 ± 1.43 | 6.23 ± 0.67 |
| 2 | 5.8 | 9.74 ± 0.14 | 11.2 ± 1.53 | 10.9 ± 1.30 |
| 3 | 4.7 | 35.9 ± 0.83 | 36.5 ± 1.15 | 35.6 ± 4.73 |
| 4 | 3.3 | 23.3 ± 0.16 | 22.2 ± 1.69 | 22.1 ± 0.39 |
| 5 | 2.1 | 7.17 ± 0.33 | 7.14 ± 0.24 | 7.08 ± 0.39 |
| 6 | 1.1 | 2.03 ± 0.21 | 2.25 ± 0.04 | 2.08 ± 0.19 |
| 7 | 0.65 | 0.74 ± 0.10 | 0.83 ± 0.04 | 0.78 ± 0.09 |
| F | 0.43 | 1.13 ± 0.27 | 0.99 ± 0.18 | 0.90 ± 0.04 |
| Entry Port | n/a | 9.10 ± 0.33 | 8.96 ± 2.77 | 9.94 ± 0.23 |
| Casings | n/a | 4.00 ± 0.88 | 4.38 ± 0.35 | 4.49 ± 0.16 |

As is evident from a comparison of particle size over time, there is a significant decrease in the change of the particle size over the course of batch manufacture (e.g., 0 to 48 hours) for the one stage process at compared to changes observed during manufacturing using the two stage fill process. Various entry ports of the Andersen Cascade Impactor may change the particle size distributions provided in the tables as is known to one of skill in the art.

**Table 7**

| The following table describes the Andersen Cascade Impactor Results for a 100 µg mometasone furoate per actuation product produced in accordance with the process of the present invention from another batch. | | | | |
|---|---|---|---|---|
| **Cascade Impactor Stage or Accessory** | **Upper Particle Size Limit µm** | **% Drug Recovery During Batch Filling** | | |
| | | **0 hours** | **24 hours** | **48 hours** |
| 0 | 10 | 0.80 ± 0.03 | 0.80 ± 0.07 | 0.79 ± 0.03 |
| 1 | 9.0 | 2.76 ± 0.10 | 2.56 ± 0.01 | 2.38 ± 0.25 |
| 2 | 5.8 | 5.07 ± 0.20 | 5.13 ± 0.24 | 5.24 ± 0.19 |
| 3 | 4.7 | 25.7 ± 0.64 | 25.7 ± 0.89 | 26.6 ± 0.46 |
| 4 | 3.3 | 34.4 ± 1.31 | 33.3 ± 2.71 | 32.1 ± 2.08 |
| 5 | 2.1 | 14.1 ± 0.68 | 12.7 ± 1.43 | 11.3 ± 1.07 |
| 6 | 1.1 | 2.68 ± 0.06 | 2.66 ± 0.17 | 2.81 ± 0.05 |
| 7 | 0.65 | 0.90 ± 0.05 | 0.96 ± 0.15 | 0.99 ± 0.03 |
| F | 0.43 | 0.86 ± 0.04 | 0.84 ± 0.03 | 0.91 ± 0.04 |
| Entry Port | n/a | 8.95 ± 1.36 | 8.42 ± 1.13 | 9.22 ± 3.25 |
| Casings | n/a | 3.68 ± 0.44 | 3.72 ± 0.39 | 3.70 ± 0.58 |

Again, as is evident from a comparison of particle size over time, there is a significant decrease in the change of the particle size over the course of batch manufacture (e.g., 0 to 48 hours) for the one stage process at compared to changes observed during manufacturing using the two stage fill process.

**Table 8 The following describes a summary of the Andersen Cascade Impactor results comparing the groupings of a mometasone furoate 100 µg/actuation Product produced by both the single stage and two stage process.**

| **Grouping** | **Cascade Impactor Stage or Accessory** | **Upper Particle Size Limit µm** | **Drug µg Recovery** | |
|---|---|---|---|---|
| | | | **2-Stage** | **1-Stage** |
| I | Entry Port, Casing | >10 | 2.7-56.5 | 29.4-38.1 |
| II | 0-2 | 4.7-10 | 29.8-84.7 | 41.6-46.2 |
| III | 3,4 | 2.1-4.7 | 57.2-124 | 91.5-101 |
| IV | 5-F | <2.1 | 13.6-24.1 | 27.5-31.8 |

As is evident, the one stage filling process eliminates particle size growth during manufacture in an improved manner over the two stage fill process, thus meeting stringent requirements for maintaining a narrower and reproducible particle size of the drug product. Indeed, with the one stage process there was obtained a tight range of particle size (91.5%-101 %) that is very desirable as opposed to the extremely broad range (57.2%-124%) for the formulations produced by the two stage process.

The foregoing descriptions of various embodiments of the invention are representative of various aspects of the invention, and are not intended to be exhaustive or limiting to the precise forms disclosed. Many modifications and variations undoubtedly will occur to those having skill in the art. It is intended that the scope of the invention shall be fully defined solely by the appended claims.

## Claims

1. A process for introducing a suspension or solution of a compound selected from the group consisting of mometasone furoate anhydrous, formoterol fumarate and combinations thereof, into a metered dose inhaler container, said container having a valve attached thereto, said process comprising the steps of:
a) introducing said compound, a surfactant and a chlorofluorocarbon free propellant into a vessel that is held under pressure to form a suspension or solution;
b) circulating said suspension or solution from the vessel through a line which includes a filling head;
c) bringing said filling head into communication with said metered dose inhaler container through said valve of said metered dose inhaler container;
d) introducing a quantity of such suspension or solution into the container from the filling head of the line through said valve of said metered dose inhaler container;
e) withdrawing said filling head from said metered dose inhaler container, and
f) sealing said metered dose inhaler container.

2. The process of claim 1, wherein said process comprises the steps of :
a) introducing said compound, a surfactant and a chlorofluorocarbon free propellant into a vessel that is held under pressure to form a suspension or solution, wherein said pressure is greater than about 30 psi;
b) circulating said suspension or solution from the vessel through a line which includes a filling head and a double diaphragm pump;
c) bringing said filling head into communication with said metered dose inhaler container through said valve of said metered dose inhaler container,
d) introducing a quantity of such suspension or solution into the container from the filling head of the line through said valve of said metered dose inhaler container;
e) withdrawing said filling head from said metered dose inhaler container; and
f) sealing said metered dose inhaler container.

3. The process of claim 1, wherein said process comprises the steps of :
a) introducing said compound a surfactant and a chlorofluorocarbon free propellant into a vessel that is held under pressure to form a suspension or solution, wherein said pressure is about 10 psi to about 15 psi;
b) circulating said suspension or solution from the vessel through a line which includes a filling head and a double diaphragm pump;
c) bringing said filling head into communication with said metered dose inhaler container through said valve of said metered dose inhaler container;
d) introducing a quantity of such suspension or solution into the container from the filling head of the line through said valve of said metered dose inhaler container;
e) withdrawing said filling head from said metered dose inhaler container; and
f) sealing said metered dose inhaler container.

4. The process of claim 1, wherein said process comprises the steps of :
a) introducing said compound, a surfactant and a chlorofluorocarbon free propellant into a vessel that is held under pressure to form a suspension or solution, wherein said pressure is about 0 psi to about 10 psi;
b) circulating said suspension or solution from the vessel through a line which includes a filling head and a single diaphragm pump;
c) bringing said filling head into communication with said metered dose inhaler container through said valve of said metered dose inhaler container;
d) introducing a quantity of such suspension or solution into the container from the filling head of the line through said valve of said metered dose inhaler container;
e) withdrawing said filling head from said metered dose inhaler container; and
f) sealing said metered dose inhaler container.

5. The process of claim 1, wherein said compound is mometasone furoate anhydrous.

6. The process of any one of claims 1-5, wherein the chlorofluorocarbon free propellant is selected from the group consisting of HFA 227 and HFA 134a.

7. The process of any one of claims 1-6, wherein the compound is micronized, and wherein at least 90% of the compound has a particle size of less than 10 µm.

8. The product produced by the process of any one of claims 1-7, wherein at least 90% of the compound has a particle size of less than 10 µm.

9. The process of claim 1, wherein said compound is a combination of mometasone furoate anhydrous and formoterol fumarate and said process comprises the steps of:
a) introducing mometasone furoate anhydrous, formoterol fumarate, a surfactant and a chlorofluorocarbon free propellant into a vessel that is held under pressure to form a suspension or solution;
b) circulating said suspension or solution from the vessel through a line which includes a filling head;
c) bringing said filling head into communication with said metered dose inhaler container through said valve of said metered dose inhaler container;
d) introducing a quantity of such suspension or solution into the container from the filling head of the line through said valve of said metered dose inhaler container;
e) withdrawing said filling head from said metered dose inhaler container; and
f) sealing said metered dose inhaler container.

10. The process of claim 9, wherein the chlorofluorocarbon free propellant is selected from the group consisting of HFA 227 and HFA 134a.

11. The process of claim 9 or 10, wherein the mometasone furoate anhydrous and formoterol fumarate are micronized, and wherein at least 90% of the mometasone furoate anhydrous and formoterol fumarate has a particle size of less than 10 µm.

12. The product produced by the process of any one of claims 9-11, wherein at least 90% of the mometasone furoate anhydrous and formoterol fumarate has a particle size of less than 10 µm.

13. The product of claim 12, wherein upon actuation of said metered dose inhaler there is dispensed about 100 µg to about 200 µg of mometasone furoate anhydrous and about 6 µg to about 12 µg of formoterol fumarate per dose.

## Patentansprüche

1. Verfahren zum Einbringen einer Suspension oder Lösung einer Verbindung ausgewählt aus der Gruppe bestehend aus Mometasonfuroat wasserfrei, Formoterolfumarat und Kombinationen davon in einen Dosierinhaliergerätebehälter, wobei der Behälter ein daran angebrachtes Ventil hat, bei dem:
a) die Verbindung, ein Tensid und ein chlorfluorkohlenstofffreies Treibmittel in ein Gefäß eingebracht werden, das unter Druck gehalten wird, um eine Suspension oder Lösung zu bilden;
b) die Suspension oder Lösung aus dem Gefäß durch eine Leitung zirkuliert wird, die einen Füllkopf einschließt;
c) der Füllkopf über das Ventil des Dosierinhaliergerätebehälters in Verbindung mit dem Dosierinhaliergerätebehälter gebracht wird;
d) eine Menge der Suspension oder Lösung aus dem Füllkopf der Leitung durch das Ventil des Dosierinhaliergerätebehälters in den Behälter eingebracht wird;
e) der Füllkopf von dem Dosierinhaliergerätebehälter abgezogen wird und
f) der Dosierinhaliergerätebehälter versiegelt wird.

2. Verfahren nach Anspruch 1, bei dem:
a) die Verbindung, ein Tensid und ein chlorfluorkohlenstofffreies Treibmittel in ein Gefäß eingebracht werden, das unter Druck gehalten wird, um eine Suspension oder Lösung zu bilden, wobei der Druck mehr als etwa 30 psi beträgt;
b) die Suspension oder Lösung aus dem Gefäß durch eine Leitung zirkuliert wird, die einen Füllkopf und eine Doppeldiaphragmapumpe einschließt;
c) der Füllkopf über das Ventil des Dosierinhaliergerätebehälters in Verbindung mit dem Dosierinhaliergerätebehälter gebracht wird;
d) eine Menge der Suspension oder Lösung aus dem Füllkopf der Leitung durch das Ventil des Dosierinhaliergerätebehälters in den Behälter eingebracht wird;
e) der Füllkopf von dem Dosierinhaliergerätebehälter abgezogen wird und
f) der Dosierinhaliergerätebehälter versiegelt wird.

3. Verfahren nach Anspruch 1, bei dem:
a) die Verbindung, ein Tensid und ein chlorfluorkohlenstofffreies Treibmittel in ein Gefäß eingebracht werden, das unter Druck gehalten wird, um eine Suspension oder Lösung zu bilden, wobei der Druck etwa 10 psi bis etwa 15 psi beträgt;
b) die Suspension oder Lösung aus dem Gefäß durch eine Leitung zirkuliert wird, die einen Füllkopf und eine Doppeldiaphragmapumpe einschließt;
c) der Füllkopf über das Ventil des Dosierinhaliergerätebehälters in Verbindung mit dem Dosierinhaliergerätebehälter gebracht wird;
d) eine Menge der Suspension oder Lösung aus dem Füllkopf der Leitung durch das Ventil des Dosierinhaliergerätebehälters in den Behälter eingebracht wird;
e) der Füllkopf von dem Dosierinhaliergerätebehälter abgezogen wird und
f) der Dosierinhaliergerätebehälter versiegelt wird.

4. Verfahren nach Anspruch 1, bei dem:
a) die Verbindung, ein Tensid und ein chlorfluorkohlenstofffreies Treibmittel in ein Gefäß eingebracht werden, das unter Druck gehalten wird, um eine Suspension oder Lösung zu bilden, wobei der Druck etwa 0 psi bis etwa 10 psi beträgt;
b) die Suspension oder Lösung aus dem Gefäß durch eine Leitung zirkuliert wird, die einen Füllkopf und eine Einzeldiaphragmapumpe einschließt;
c) der Füllkopf über das Ventil des Dosierinhaliergerätebehälters in Verbindung mit dem Dosierinhaliergerätebehälter gebracht wird;
d) eine Menge der Suspension oder Lösung aus dem Füllkopf der Leitung durch das Ventil des Dosierinhaliergerätebehälters in den Behälter eingebracht wird,
e) der Füllkopf von dem Dosierinhaliergerätebehälter abgezogen wird und
f) der Dosierinhaliergerätebehälter versiegelt wird.

5. Verfahren nach Anspruch 1, bei dem die Verbindung Mometasonfuroat wasserfrei ist.

6. Verfahren nach einem der Ansprüche 1 bis 5, bei dem das chlorfluorkohlenstofffreie Treibmittel aus der Gruppe bestehend aus HFA 227 und HFA 134a ausgewählt ist.

7. Verfahren nach einem der Ansprüche 1 bis 6, bei dem die Verbindung mikronisiert ist und mindestens 90 % der Verbindung eine Teilchengröße von weniger als 10 µm haben.

8. Produkt, das nach dem Verfahren gemäß einem der Ansprüche 1 bis 7 hergestellt ist, wobei mindestens 90 % der Verbindung eine Teilchengröße von weniger als 10 µm haben.

9. Verfahren nach Anspruch 1, bei dem die Verbindung eine Kombination von Mometasonfuroat wasserfrei und Formoterolfumarat ist und bei dem
a) Mometasonfuroat wasserfrei, Formoterolfumarat, ein Tensid und ein chlorfluorkohlenstofffreies Treibmittel in ein Gefäß eingebracht werden, das unter Druck gehalten wird, um eine Suspension oder Lösung zu bilden;
b) die Suspension oder Lösung aus dem Gefäß durch eine Leitung zirkuliert wird, die einen Füllkopf einschließt;
c) der Füllkopf über das Ventil des Dosierinhaliergerätebehälters in Verbindung mit dem Dosierinhaliergerätebehälter gebracht wird;
d) eine Menge der Suspension oder Lösung aus dem Füllkopf der Leitung durch das Ventil des Dosierinhaliergerätebehälters in den Behälter eingebracht wird;
e) der Füllkopf von dem Dosierinhaliergerätebehälter abgezogen wird und
f) der Dosierinhaliergerätebehälter versiegelt wird.

10. Verfahren nach Anspruch 9, bei dem das chlorfluorkohlenstofffreie Treibmittel aus der Gruppe bestehend aus HFA 227 und HFA 134a ausgewählt ist.

11. Verfahren nach Anspruch 9 oder 10, bei dem das Mometasonfuroat wasserfrei und Formoterolfumarat mikronisiert sind und mindestens 90 % des Mometasonfuroats, wasserfrei, und des Formoterolfumarats eine Teilchengröße kleiner als 10 µm haben.

12. Produkt, das nach dem Verfahren gemäß einem der Ansprüche 9 bis 11 hergestellt ist, wobei mindestens 90 % des Mometasonfuroats, wasserfrei, und Formoterolfumarats eine Teilchengröße von weniger als 10 µm haben.

13. Produkt nach Anspruch 12, bei dem nach Betätigung des Dosierinhaliergeräts etwa 100 µg bis etwa 200 µg Mometasonfuroat wasserfrei und etwa 6 µg bis etwa 12 µg Formoterolfumarat pro Dosis abgegeben werden.

## Revendications

1. Procédé d'introduction, dans le réceptacle d'un inhalateur-doseur, d'une suspension ou d'une solution d'un composé choisi parmi du furoate de mométasone anhydre, du fumarate de formotérol et leurs combinaisons, ledit réceptacle étant équipé d'une vanne, lequel procédé comporte les étapes suivantes :
a) introduire ledit composé, un tensioactif et un agent propulseur sans chlorofluorocarbure dans un récipient où est maintenue une certaine pression, pour en faire une suspension ou une solution ;
b) faire circuler ladite suspension ou solution, depuis le récipient, dans un conduit qui comporte une tête de remplissage ;
c) mettre ladite tête de remplissage en communication avec ledit réceptacle d'inhalateur-doseur, par l'intermédiaire de ladite vanne dudit réceptacle d'inhalateur-doseur ;
d) introduire dans le réceptacle une certaine quantité de ladite suspension ou solution, depuis ladite tête de remplissage du conduit, par ladite vanne dudit réceptacle d'inhalateur-doseur ;
e) retirer ladite tête de remplissage dudit réceptacle d'inhalateur-doseur ;
f) et fermer hermétiquement ledit réceptacle d'inhalateur-doseur.

2. Procédé conforme à la revendication 1, lequel procédé comporte les étapes suivantes :
a) introduire ledit composé, un tensioactif et un agent propulseur sans chlorofluorocarbure dans un récipient où est maintenue une certaine pression, pour en faire une suspension ou une solution, ladite pression étant supérieure à environ 207 kPa (30 psi) ;
b) faire circuler ladite suspension ou solution, depuis le récipient, dans un conduit qui comporte une tête de remplissage et une pompe à double diaphragme ;
c) mettre ladite tête de remplissage en communication avec ledit réceptacle d'inhalateur-doseur, par l'intermédiaire de ladite vanne dudit réceptacle d'inhalateur-doseur ;
d) introduire dans le réceptacle une certaine quantité de ladite suspension ou solution, depuis ladite tête de remplissage du conduit, par ladite vanne dudit réceptacle d'inhalateur-doseur ;
e) retirer ladite tête de remplissage dudit réceptacle d'inhalateur-doseur ;
f) et fermer hermétiquement ledit réceptacle d'inhalateur-doseur.

3. Procédé conforme à la revendication 1, lequel procédé comporte les étapes suivantes :
a) introduire ledit composé, un tensioactif et un agent propulseur sans chlorofluorocarbure dans un récipient où est maintenue une certaine pression, pour en faire une suspension ou une solution, ladite pression valant d'environ 69 kPa (10 psi) à environ 104 kPa (15 psi) ;
b) faire circuler ladite suspension ou solution, depuis le récipient, dans un conduit qui comporte une tête de remplissage et une pompe à double diaphragme ;
c) mettre ladite tête de remplissage en communication avec ledit réceptacle d'inhalateur-doseur, par l'intermédiaire de ladite vanne dudit réceptacle d'inhalateur-doseur ;
d) introduire dans le réceptacle une certaine quantité de ladite suspension ou solution, depuis ladite tête de remplissage du conduit, par ladite vanne dudit réceptacle d'inhalateur-doseur ;
e) retirer ladite tête de remplissage dudit réceptacle d'inhalateur-doseur ;
f) et fermer hermétiquement ledit réceptacle d'inhalateur-doseur.

4. Procédé conforme à la revendication 1, lequel procédé comporte les étapes suivantes :
a) introduire ledit composé, un tensioactif et un agent propulseur sans chlorofluorocarbure dans un récipient où est maintenue une certaine pression, pour en faire une suspension ou une solution, ladite pression valant d'environ 0 kPa (0 psi) à environ 69 kPa (10 psi) ;
b) faire circuler ladite suspension ou solution, depuis le récipient, dans un conduit qui comporte une tête de remplissage et une pompe à simple diaphragme ;
c) mettre ladite tête de remplissage en communication avec ledit réceptacle d'inhalateur-doseur, par l'intermédiaire de ladite vanne dudit réceptacle d'inhalateur-doseur ;
d) introduire dans le réceptacle une certaine quantité de ladite suspension ou solution, depuis ladite tête de remplissage du conduit, par ladite vanne dudit réceptacle d'inhalateur-doseur ;
e) retirer ladite tête de remplissage dudit réceptacle d'inhalateur-doseur ;
f) et fermer hermétiquement ledit réceptacle d'inhalateur-doseur.

5. Procédé conforme à la revendication 1, dans lequel ledit composé est du furoate de mométasone anhydre.

6. Procédé conforme à l'une des revendications 1 à 5, dans lequel l'agent propulseur sans chlorofluorocarbure est choisi dans l'ensemble que constituent le HFA-227 et le HFA-134a.

7. Procédé conforme à l'une des revendications 1 à 6, dans lequel le composé est micronisé, à tel point qu'au moins 90 % du composé se présente en particules de taille inférieure à 10 µm.

8. Produit fourni par un procédé conforme à l'une des revendications 1 à 7, dans lequel au moins 90 % du composé se présente en particules de taille inférieure à 10 µm.

9. Procédé conforme à la revendication 1, dans lequel ledit composé est une combinaison de furoate de mométasone anhydre et de fumarate de formotérol, et lequel procédé comporte les étapes suivantes :
a) introduire du furoate de mométasone anhydre, du fumarate de formotérol, un tensioactif et un agent propulseur sans chlorofluorocarbure dans un récipient où est maintenue une certaine pression, pour en faire une suspension ou une solution ;
b) faire circuler ladite suspension ou solution, depuis le récipient, dans un conduit qui comporte une tête de remplissage ;
c) mettre ladite tête de remplissage en communication avec ledit réceptacle d'inhalateur-doseur, par l'intermédiaire de ladite vanne dudit réceptacle d'inhalateur-doseur ;
d) introduire dans le réceptacle une certaine quantité de ladite suspension ou solution, depuis ladite tête de remplissage du conduit, par ladite vanne dudit réceptacle d'inhalateur-doseur ;
e) retirer ladite tête de remplissage dudit réceptacle d'inhalateur-doseur ;
f) et fermer hermétiquement ledit réceptacle d'inhalateur-doseur.

10. Procédé conforme à la revendication 9, dans lequel l'agent propulseur sans chlorofluorocarbure est choisi dans l'ensemble que constituent le HFA-227 et le HFA-134a.

11. Procédé conforme à la revendication 9 ou 10, dans lequel le furoate de mométasone anhydre et le fumarate de formotérol sont micronisés, à tel point qu'au moins 90 % du furoate de mométasone anhydre et du fumarate de formotérol se présentent en particules de taille inférieure à 10 µm.

12. Produit fourni par un procédé conforme à l'une des revendications 9 à 11, dans lequel au moins 90 % du furoate de mométasone anhydre et du fumarate de formotérol se présentent en particules de taille inférieure à 10 µm.

13. Produit conforme à la revendication 12, qui délivre, lorsqu'on actionne ledit inhalateur-doseur, d'environ 100 µg à environ 200 µg de furoate de mométasone anhydre et d'environ 6 µg à environ 12 µg de fumarate de formotérol par dose.
